# EUROPEAN PATENT APPLICATION

(11) **EP 1 679 057 A1**
(43) Date of publication of application: **12.07.2006**
(21) Application number: 04792116.8
(22) Date of filing: 07.10.2004
(51) Int. Cl.: A61J 1/05

(54) **CONTAINER MANUFACTURING DEVICE**

(30) Priority: 07.10.2003 JP 2003348103
(71) Applicant: SANTEN PHARMACEUTICAL CO., LTD., Higashiyodogawa-ku, Osaka-shi, Osaka 533-8651 (JP)
(72) Inventor: KUSU, Yukio, SANTEN PHARMACEUTICAL CO., LTD., Osaka-shi, Osaka 5338651 (JP)
(74) Representative: Brommer, Hans Joachim
(86) International application number: PCT/JP2004/014818
(87) International publication number: WO 2005/034836

(57) **Abstract**

A container manufacturing apparatus for manufacturing a container body made of a thermoplastic material having a liquid storage portion for containing chemical liquid therein and an instilling hole for controlling a drop amount of discharged chemical liquid to be a predetermined amount, comprising a piercing unit X for piercing the container body to form the instilling hole therein, wherein the piercing unit X includes a needle-like portion 10 for forming the instilling hole, and a PTC heater 11 for heating the needle-like portion 10.

## Description

### TECHNICAL FIELD

The present invention relates to a container manufacturing apparatus for manufacturing a container body made of a thermoplastic material having a liquid storage portion for containing liquid therein and an instilling hole for controlling a drop amount of discharged liquid to be a predetermined amount, the apparatus comprising a piercing unit for piercing the container body to form the instilling hole therein.

### BACKGROUND ART

It is required in medical eyedrops that a drop amount be controlled to be a predetermined amount. In order to perform such control, an eyedrops container provided with an inner plug forming an instilling hole therein is in wide use.
On the other hand, an integral molding type eyedrops container dispensing with the inner plug and having a container body integrally formed with the instilling hole has been known.
In the integral molding type eyedrops container, the container body is made of a thermoplastic material and loaded with liquid in a sealed condition simultaneously with blow molding or vacuum forming (what is called a bottle pack container body). The container body includes a male screw portion formed on an outer periphery of a tip end thereof. A cap is removably screwed on the male screw portion, the cap having a needle-like projection (piercing member) integrally formed therewith for piercing the tip end of the container body to form the instilling hole. The instilling hole is formed by the needle-like projection of the cap piercing the tip end of the container body when the cap is screwed in a fastening direction one step deeper than a normal stop position of the cap.

In such an eyedrops container, since the instilling hole is formed while the needle-like projection of the cap is piercing the tip end of the container body, the shape and size of the instilling hole could become uneven unless the amount of operation is adequate in screwing the cap in the fastening direction from the normal stop position. This may incur the possibility of varying the drop amount of liquid discharged from the container body.
On the other hand, when the cap is screwed excessively in the fastening direction from the normal stop position after the instilling hole is formed in the tip end of the container body, the instilling hole is expanded by the needle-like projection of the cap every time an excessive fastening operation is executed. This may lead to a possibility of gradually increasing the drop amount of liquid discharged from the container body.

In order to overcome these drawbacks, a container is known in which the instilling hole is formed by a method of piercing the tip end of an instilling tube with a needle-like mold in manufacturing the container body without using the needle-like projection provided on the cap.
For instance, Patent Document 1 discloses a method using a needle-like mold under a room temperature condition or a heated temperature condition, while Patent Document 2 discloses a method of using a heated needle (needle-like mold).
Patent Document 1: JP Patent Publication "Kokai" No. 2001-120639 (see paragraph 0029)
Patent Document 2: JP U.M. Publication No. S35-10375 (see page 1 and Fig. 3)

### DISCLOSURE OF THE INVENTIN

### PROBLEM TO BE SOLVED BY THE INVENTION

As noted above, when the needle-like projection of the cap acting as an unheated needle is used in forming an instilling hole, variations occur in an opening area of the instilling hole, or the instilling hole may be shaped like a slit, i.e. a disadvantage of increased oblateness of the area of the instilling hole.

In view of this, the needle-like mold heated by heating means such as high frequency induction heating technique, a halogen lamp, hot air or the like as disclosed in Patent Document 1 is used, or the needle-like mold heated by flames as disclosed in Patent Document 2 is used, to form the instilling hole. As a result, instilling holes of generally uniform size are formed corresponding to the diameter of the needle.

However, it is difficult to control the temperature of the needle tip by the high frequency induction heating technique, halogen lamp, hot air, flames or the like as described above. In other words, there is a problem of difficulty in determining a proper temperature for the needle tip to form the instilling hole.

Therefore, the object of the present invention is to provide a container manufacturing apparatus including a piercing unit for readily controlling temperature, and capable of forming instilling holes having an opening area with less variations and a shape with reduced oblateness and close to a circle.

### MEANS FOR SOLVING THE PROBLEM

In order to fulfill the above-noted object, a first characteristic feature of a container manufacturing apparatus according to the present invention lies in the apparatus for manufacturing a container body made of a thermoplastic material having a liquid storage portion for containing liquid therein and an instilling hole for controlling a drop amount of discharged liquid to be a predetermined amount, comprising a piercing unit for piercing the container body to form the instilling hole therein, wherein the piercing unit includes a needle-like portion for forming the instilling hole, and a PTC heater for heating the needle-like portion.

The PTC heater generates heat by itself due to Joule heat when supplied with electricity through electrodes. The resistance is increased logarithmically when temperature exceeds Curie temperature (Tc). Then, the heat generating temperature is reduced since the electric current diminishes to restrain electric power. At this time, resistance is reduced and thus the electric current is increased. As a result, the electric power is increased to raise the heat generating temperature. By repeating this cycle, the PTC heater acts as a fixed temperature heat generator having a self-control function.
As the needle-like portion is arranged in the proximity of the PTC heater, the heat is conducted from the self-heated PTC heater to the needle-like portion.
It should be noted that the heat generating temperature is substantially constant because the PTC heater is the fixed temperature heat generator having a self-control function for temperature. In other words, the heat amount conducted from the PTC heater to the needle-like portion is considered substantially constant as well. Thus, the temperature of the needle-like portion can easily be maintained constant.

Hence, the container manufacturing apparatus defined in the first characteristic feature of the present invention can provide the piercing unit having the needle-like portion for easily maintaining a proper temperature for forming the instilling hole by controlling the PTC heater at a proper temperature for forming the instilling hole.
Since this piercing unit includes the needle-like portion acting as a heated needle whose temperature can be controlled easily, it is possible to form the instilling hole having an opening area with less variations and a shape with reduced oblateness that is close to a circle. The needle-like portion may maintain the proper temperature almost constantly for forming the instilling hole when the PTC heater is controlled to the proper temperature for forming the instilling hole.

A second characteristic feature of a container manufacturing apparatus according to the present invention lies in that the PTC heater surrounds the needle-like portion.

According to the container manufacturing apparatus defined in the second characteristic feature of the present invention, the heat of the PTC heater generated by electrification is efficiently conducted to the needle-like portion.

A third characteristic feature of a container manufacturing apparatus according to the present invention lies in that the PTC heater is arranged not to contact the needle-like portion directly.

According to the container manufacturing apparatus defined in the third characteristic feature of the present invention, the needle-like portion is not electrified even if the PTC heater is electrified because the PTC heater is not in direct contact with the needle-like portion. This is effective to prevent a failure of the container manufacturing apparatus caused by electric leakage, inadvertent variations of a predetermined temperature, and so on.

### BEST MODE FOR CARRYING OUT THE INVENTION

An embodiment of the present invention will be described hereinafter with reference to the accompanying drawings.
A container manufacturing apparatus according to the present invention can be used for the purpose of piercing an eyedrops container mainly for medical use to form an instilling hole through which medical liquid is discharged, for example.

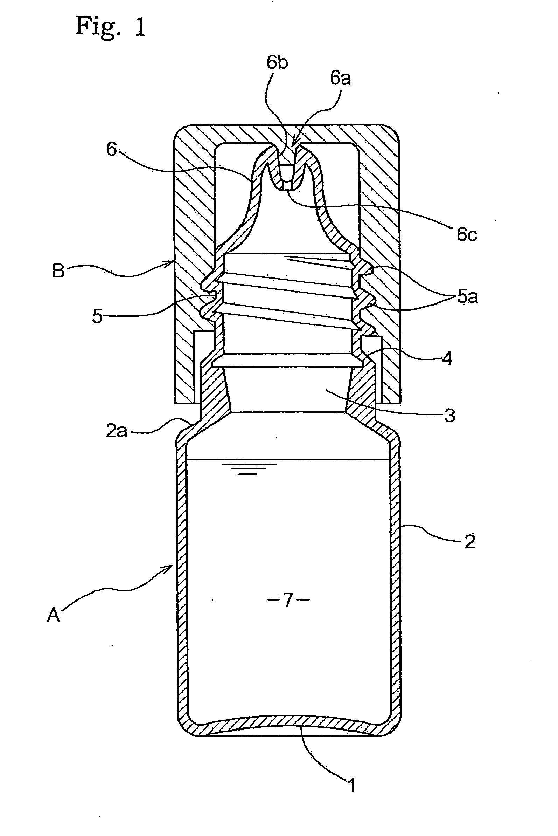

As shown in Fig. 1, the eyedrops container comprises a container body A made of a flexible thermoplastic material and filled with a predetermined amount of medical liquid simultaneously with blow molding or vacuum forming, and a cap B removably screwed to a male screw portion 5a formed on an outer peripheral surface of a threaded tube 5 of the container body A.
The container body A includes a liquid storage portion 7 for containing the liquid therein, a circular bottom 1 curved inwardly, a barrel 2 in the form of a hollow cylinder continuous with a periphery of the bottom, a cylindrical neck portion 3 formed continuous from a shoulder portion 2a of the barrel 2, an annular stepped portion 4 bulging radially outwardly from an upper position of the neck portion 3, the threaded tube 5 with the male screw portion 5a continuous upward from the stepped portion, and an instilling tube 6 continuous upward from the threaded tube.
The instilling tube 6 has an instilling hole 6c for controlling a drop amount of the liquid to be discharged to be a predetermined amount.

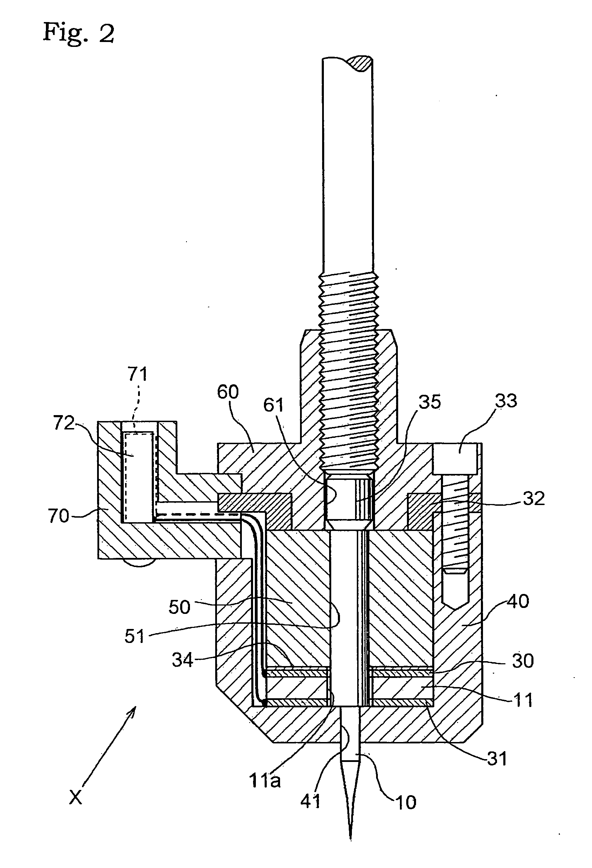

The container manufacturing apparatus includes a piercing unit for forming the instilling hole 6c in manufacturing the container body A.
As shown in Fig. 2, the piercing unit X has a needle-like portion 10 for forming the instilling hole 6c, and a PTC heater 11 for heating the needle-like portion 10. The piercing unit X will be described in detail hereinafter.

The needle-like portion 10 is shaped to be sharp-pointed having a diameter decreasing toward a tip end thereof, for example, to facilitate forming of the instilling hole 6c. More specifically, the diameter of the tip end is preferably within the range of 0.1mm through 0.8mm. The tip end projects outward through a bore 41 of a holder 40 housing the needle-like portion 10.
The needle-like portion 10 extends through bores 51 and 61 formed, respectively, in a core 50 mounted in the holder 40 and in a cover 60 for sealing the holder 40. A flat-point hollow set screw 35 is provided in an intermediate position of the needle-like portion 10 to contact the inner wall of the bore 61. The cover 60 is fixed to the holder 40 by a cap bolt 33 through an insulating collar 32.

The needle-like portion 10 is formed of SUS316, for example. It is desirable to apply a DLC (diamond-like carbon) coating to the surface at the tip end of the needle-like portion 10.
The DLC coating can provide excellent surface smoothness and good friction and abrasion properties, and thus can be used effectively as a lubricating anti-abrasion film as well as aiming at an extended tool life. Further, the surface smoothness can improve peelability and mould-releasability of the resin, thereby to promote operational efficiency.

The PTC heater 11 represents a heater using a PTC thermistor (Positive Temperature Coefficient Thermistor). It is preferably accommodated in the holder 40 to be close to the needle-like portion 10.
The PTC thermistor comprises semiconductor ceramics containing barium titanate (BaTiO₃) as a main component, and may set Curie temperature (Tc) as desired by selecting a material composition. Having a nature of rapidly increasing the electric resistance when reaching Curie temperature, the PTC thermister is used as a fixed temperature heat generator.

The PTC heater 11 may have any of various shapes including a disk shape, rectangular plate shape, ring shape, chip shape and the like. The present embodiment employs a ring-shaped thermistor having a bore 11a formed therein. The needle-like portion 10 extends through the bore 11a.

The PTC heater 11 has a top surface contacting a top surface electrode 30 and a bottom surface contacting a bottom surface electrode 31, respectively, and is fixed to the core 50 through the top surface electrode 30 and a washer 34.
A connector case 70 is attached to the holder 40 and the cover 60, and includes two lead wires and terminals 71 and 72 provided inside thereof. Electric connection can be established between the top surface electrode 30 and the terminal 71, and between the bottom surface electrode 31 and the terminal 72 through the lead wires, respectively. Each of the terminals 71 and 72 is connectable to an external power source.
The PTC heater 11 is operable by rated voltages such as AC100V, AC200V, DC12V, DC24V and the like, for example.

The PTC heater 11 is controlled preferably to 70 through 120°C. The temperature is controlled by supplying the PTC heater 11 with electricity from the external power source through the terminals 71 and 72, the lead wires, and the electrodes 30 and 31. More particularly, when both of the electrodes 30 and 31 are electrified, a voltage is applied to the PTC heater 11 contacting these electrodes at the top and bottom surfaces thereof. Then, the PTC heater 11 generates heat by itself due to Joule heat. When the heater exceeds Curie temperature (Tc), the value of resistance is logarithmically increased. Then, the electric current is reduced to restrain electric power and lower the heat-generating temperature. At this point, since the value of resistance is diminished, the electric current is increased. As a result, electric power is increased to raise the heat-generating temperature. The PTC heater 11 acts as a fixed temperature heat generator having a self-control function by repeating this cycle.

Thus, the piercing unit X includes the needle-like portion 10 having the instilling hole 6c formed therein, and the PTC heater 11 acting as the fixed temperature heat generator.
As noted above, the PTC heater is supplied with electricity from the external power source through both of the electrodes 30 and 31 during a piercing operation for forming the instilling hole 6c. At this time, the PTC heater 11 generates heat which is conducted to the needle-like portion 10 arranged in the proximity of the heater.
Since the PTC heater 11 is the fixed temperature heat generator having a self-control function for temperature, the heat generating temperature is substantially constant. Therefore, the heat amount conducted from the PTC heater 11 to the needle-like portion 10 is substantially constant as well, whereby the temperature of the needle-like portion 10 can be easily maintained constant.
Thus, the present invention can provide the piercing unit X having the needle-like portion 10 that can easily maintain a proper temperature for forming the instilling hole 6c by controlling the PTC heater 11 to a proper temperature for forming the instilling hole 6c during a piercing operation to form the instilling hole 6c.
More particularly, this piercing unit X has the needle-like portion 10 acting as a heated needle whose temperature is easy to control, and thus can form instilling holes 6c having their opening area with less variations. The needle-like portion 10 can almost constantly maintain a proper temperature for forming the instilling hole 6c when the PTC heater 11 is controlled to a proper temperature for forming the instilling hole 6c.

It is preferable that the PTC heater 11 is formed to surround the needle-like portion 10.
In the present embodiment, the needle-like portion 10 extends through the bore 11a of the ring-shaped PTC heater 11, and the inner wall of the bore 11a surrounds the needle-like portion 10. In such a construction, the heat generated from the PTC heater 11 supplied with electricity can be conducted to the needle-like portion 10 efficiently.

It is also preferable to provide the PTC heater 11 so as not to directly contact the needle-like portion 10.
In this case, the needle-like portion10 may be 4.0mm in diameter while the bore 11a of the PTC heater 11 may be 4.3mm in diameter, for example.
Although the PTC heater 11 is supplied with electricity from the external power source through both of the electrodes 30 and 31 during an operation to form the instilling hole 6c, the needle-like portion 10 is not electrified since the PTC heater 11 is not in direct contact with the needle-like portion 10. This is effective to prevent a failure of the container manufacturing apparatus caused by electric leakage, inadvertent variations of a predetermined temperature, and so on.

On the other hand, the PTC heater 11, though not in direct contact with the needle-like portion 10, can heat the needle-like portion 10 since radiant heat of the PTC heater 11 under temperature control by electrification is conducted to the needle-like portion 10.

Among the components noted above, the insulating collar 32, holder 40, core 50, connector case 70 and connector cover 73 are, preferably but not limitatively, made of polyether ether ketone (PEEK) having excellent heat-resistant property, strength and insulating property.
The core 50 and connector cover 73 formed of the good heat-resisting material as above may have little chance of being deformed or broken, even if the needle-like portion 10 extending through the bores 51 and 61 formed in these components maintains heat enough to form the instilling hole 6c.
It should be noted that the top surface electrode 30, bottom surface electrode 31, cap bolt 33, washer 34, flat-point hollow set screw 35, cover 60 and trussed screw 74 are, preferably but not limitatively, made of SUS304.

The container manufacturing apparatus comprises various units usually required for manufacturing the eyedrops container, including a container body manufacturing unit for manufacturing the container body A loaded with the medical liquid in a sealed condition simultaneously with formation by blow molding or vacuum forming technique, a forming unit having an instilling opening forming device and the piercing unit X noted above for forming an instilling opening 6a in the container body A, a transport and supply unit for transporting the container body A made by blow molding or vacuum forming technique to the forming unit, a delivery unit for transporting the container body A having the instilling hole 6c formed therein by the piercing unit X to a location of the process for labeling and packaging the container body A, and so on.

### EMBODIMENT 1

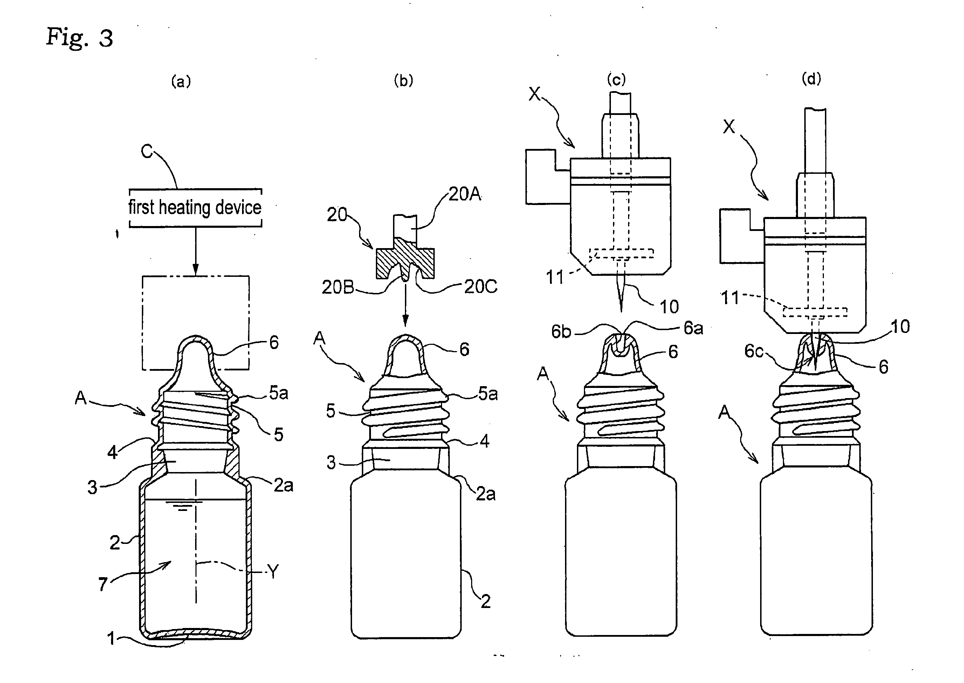

A process for piercing the above-noted eyedrops container to form the instilling hole 6c therein using the container manufacturing apparatus according to the present invention will be described hereinafter (see Fig. 3).

The thermoplastic material constituting the container body A may be any of polyethylene, polyethylene-polypropylene, polypropylene, polyethylene-terephthalate, polycarbonate and so on. In this embodiment, the container body A made of polyethylene will be described.

The container body A is manufactured by blow molding or vacuum forming technique in the container body manufacturing unit noted above. The blow molding or vacuum forming can be performed by a general process. The formed container body A is transported to the forming unit by the transport and supply unit.

In the forming unit, the instilling opening 6a (see Fig. 1) is formed in the container body A by the instilling opening forming unit.
More particularly, as shown in Fig. 3(a), the instilling tube 6 forming the tip end of the container body A is heated in part to room temperature or 70°C through 150°C by a first heating device C such as hot air, a halogen lamp, laser beam or the like. A preferred heating temperature depends on the material or shape of the container body A, but may be selected so that the tip end of the container body A is somewhat softened.

Next, as shown in Fig. 3(b), a convex mold 20 acting as the instilling opening forming unit is pressed in an axial direction Y of the container to the container body A before cooling of the instilling tube 6 of the container body A heated by the first heating device C. Thus, a bottomed conical recess 6b (see Fig. 1) is formed in the instilling tube 6 of the container body A to have a larger inner diameter toward the instilling opening 6a.
The convex mold 20 includes a mounting shaft 20A defining, at a distal end thereof, a conical molding projection 20B for forming the bottomed conical recess 6b, and a bowl-shaped (bell-shaped) molding surface 20C for forming an outer periphery of the instilling tube 6 of the container body A.

The recess 6b has a depth in a range of 2 through 7mm, preferably in a range of 5 through 7mm, and most preferably 6mm. The opening diameter (mouth diameter) of the instilling opening 6a is adjusted within a range of 2.0 through 4.0mm depending on the liquidity of the medical liquid (surface tension and viscosity). In order to fix a drop amount (to be adjusted within a range of 25 through 50µL per drop depending on purpose), the diameter of the instilling opening 6a is reduced for a liquid of high surface tension while the diameter of the instilling opening 6a is increased for a liquid of low surface tension.

After the instilling opening 6a is formed in the container body A, the instilling hole 6c is formed in the container body A by the piercing unit X.

More particularly, as shown in Figs. 3(c) and (d), the piercing unit X is pressed to a middle portion of the bottom of the recess 6b formed in the instilling tube 6 of the container body A from the axial direction Y of the container body, thereby to form the instilling hole 6c.
At this time, the PTC heater 11 is supplied with electricity from the external power source to generate heat which is conducted to the needle-like portion 10 adjacent thereto. The PTC heater 11 is heated to each of the temperatures shown in Table 1 below, for example. Since the temperature of the heat generated by the PTC heater 11 acting as the fixed temperature heat generator is substantially constant, the heat amount conducted form the PTC heater 11 to the needle-like portion 10 is also substantially constant. The temperature of the needle-like portion 10 is maintained constant around each of the temperature. The instilling holes 6c are formed at the respective temperatures.

The instilling hole 6c is formed using the needle-like portion 10 of which the diameter of the tip end is, for example, within a range of 0.1 through 0.8mm. The smaller diameter of the needle-like portion is the more preferable, and the most preferable size is approximately 0.2mm in diameter. On the other hand, since it is technically hard to form a needle with an extremely small diameter, the needle having a range of 0.4 through 0.6mm in diameter is actually used.

The processed container body A having the instilling hole 6c formed therein is labeled and packaged to eventually manufacture the eyedrops container.

In order to evaluate the instilling hole 6c of the eyedrops container formed in this way, oblateness was measured. The measurement was performed by measuring oblateness of the instilling holes 6c formed in the temperatures shown in Table 1, respectively, when the PTC heater 11 was not electrified (at room temperature), and when the PTC heater 11 was heated to temperatures in the range of 30 through 140°C as shown in Table 1, respectively. Ten instilling holes 6c were formed at each temperature and the oblateness thereof was measured. Average values of oblateness in the respective temperatures are shown in Table 1.
It should be noted that the oblateness represents values obtained by (shorter diameter/longer diameter) in the instilling hole 6c x 100.

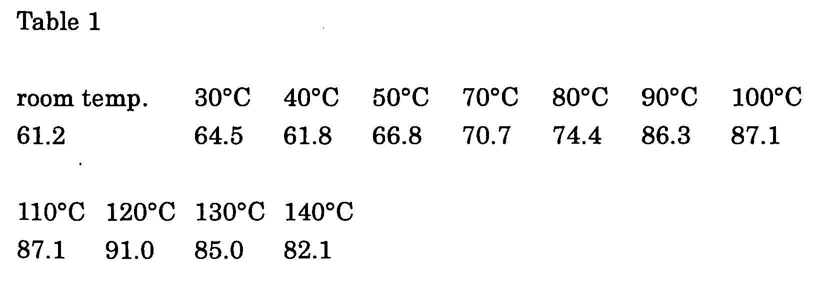

The results prove that good results of oblateness of 70 or more are obtained when the PTC heater 11 is heated to 70°C or higher.
It has also turned out that the resin is sometimes hard to peel off from the tip end of the needle-like portion 10 when the heating temperature is 130°C or higher. Thus, the heating temperature of the PTC heater 11 is preferably in the range of 70 through 120°C. Further, the range of 100 through 120°C is most preferable in view of the obtained values of oblateness.

### INDUSTRIAL UTILITY

The container manufacturing apparatus according to the present invention may be used for the purpose of piercing the eyedrops container mainly for medical use to form the instilling hole through which the medical liquid is discharged, for example.

### BRIEF DESCRIPTION OF THE DRAWINGS

[Fig. 1] Schematic view of an eyedrops container.
[Fig. 2] Schematic view of a piercing unit of a container manufacturing apparatus according to the present invention.
[Fig. 3] Schematic view of a process of piercing the eyedrops container to form an instilling hole using the container manufacturing apparatus according to the present invention.

### DESCRIPTION OF THE REFERENCE SIGNS

- 6c: instilling hole
- 7: liquid storage portion
- 10: needle-like portion
- 11: PTC heater
- A: container body
- X: piercing unit

## Claims

1. A container manufacturing apparatus for manufacturing a container body made of a thermoplastic material having a liquid storage portion for containing liquid therein and an instilling hole for controlling a drop amount of discharged liquid to be a predetermined amount, comprising a piercing unit for piercing the container body to form the instilling hole therein,
wherein the piercing unit includes a needle-like portion for forming the instilling hole, and a PTC heater for heating the needle-like portion.

2. A container manufacturing apparatus as claimed in Claim 1, wherein the PTC heater surrounds the needle-like portion.

3. A container manufacturing apparatus as claimed in Claim 1 or 2, wherein the PTC heater is arranged not to contact the needle-like portion directly.
